Europäisches Patentamt

European Patent Office

Office européen des brevets

(11) Numéro de publication: **0 108 658**

**A1**

(12) **DEMANDE DE BREVET EUROPEEN**

(21) Numéro de dépôt: **83401872.3**

(22) Date de dépôt: **23.09.83**

(51) Int. Cl.³: **A 61 M 1/03**
**A 61 K 35/14**

(30) Priorité: **24.09.82 FR 8216153**

(43) Date de publication de la demande:
**16.05.84 Bulletin 84/20**

(84) Etats contractants désignés:
**AT BE CH DE FR GB IT LI LU NL SE**

(71) Demandeur: **INSTITUT NATIONAL DE LA SANTE ET DE LA RECHERCHE MEDICALE (INSERM)**
**101, rue de Tolbiac**
**F-75654 Paris Cedex 13(FR)**

(72) Inventeur: **Rivat, Claude**
**37, Allée de Fribourg Av. des 4 Cantons**
**76000 Rouen(FR)**

(72) Inventeur: **Larue, Catherine**
**3, rue E. Galylaire**
**76290 Fontaine-La-Mallet(FR)**

(74) Mandataire: **Ayache, Monique et al,**
**CABINET PLASSERAUD 84, rue d'Amsterdam**
**F-75009 Paris(FR)**

(54) **Dispositif pour l'élimination ou la récupération d'anticorps ou d'antigènes à partir du sang, son obtention et son utilisation.**

(57) Le dispositif (5) est essentiellement constitué d'un faisceau de fibres creuses capillaires, de préférence des fibres de cellulose ou de copolymère d'alcool éthylène-vinylique, à l'intérieur desquelles est fixé par covalence un ligand sélectif pour la substance, anticorps ou antigène, à éliminer ou récupérer, présente dans le sang. Pour purifier le sang par circulation extra-corporelle "in vivo", on le prélève par exemple dans l'artère fémorale (1), on ajoute l'héparine (3) avant de le faire passer à travers le dispositif (5) puis on le dégaze (7) et le renvoie dans la veine fémorale (10).

EP 0 108 658 A1

Dispositif pour l'élimination ou la récupération d'anticorps ou
d'antigènes à partir du sang, son obtention et son utilisation.

L'invention concerne l'élimination ou la récupération de substances biologiquement·actives à partir du sang.

Elle a plus particulièrement pour objet un dispositif permettant d'éliminer du sang, de façon spécifique, en tant que constituants indésirables des anticorps ou des antigènes "in vitro" et surtout par circulation extracorporelle "in vivo", sans qu'il soit nécessaire d'effectuer au préalable la séparation du plasma et des cellules sanguines, ou d'en extraire des substances biologiquement actives, anticorps ou antigènes, recherchées notamment en thérapeutique.

L'invention a, en outre, pour objet des procédés de traitement du sang mettant en oeuvre ce dispositif.

La mise au point de procédés d'élimination spécifique de certaines substances à partir du sang, notamment l'élimination "in vivo" d'anticorps circulants, préoccupe depuis un certain nombre d'années un nombre croissant d'immunologistes. En effet, dans certains troubles immunitaires, le rôle d'anticorps ou de complexes antigènes-anticorps pathogènes semble démontré. Dans un certain nombre d'autres cas, la présence d'anticorps peut se révéler néfaste pendant certaines périodes particulières et il serait extrêmement utile de pouvoir les éliminer ou,‾tout au·moins, maintenir leur taux à une valeur basse.

Des méthodes thérapeutiques ont été proposées comme l'immunosuppression et plus récemment l'immunostimulation et les méthodes de soustraction plasmatique. Aucune de ces méthodes ne s'est révélée satisfaisante, car toutes présentent des inconvénients importants.

Ainsi, l'immunosuppression non spécifique donne des résultats inconstants et des effets secondaires importants, tandis que l'immunostimulation est incertaine. Compte tenu de ces faits, on a développé des thérapeutiques de soustraction plasmatique comme les plasmaphérèses qui se sont révélées efficaces dans le traitement d'affections auto-immunes comme le lupus érythémateux disséminé, le syndrôme de Goodpasture, et plus particulièrement la myasthénie. Ces thérapeutiques sont toutefois techniquement lourdes et coûteuses. Elles ne sont pas dénuées de dangers puisqu'elles exposent le patient au risque d'infection par le virus de l'hépatite et à certaines complications hémodynamiques parfois mortelles, et en outre ne permettent pas de discerner précisément les mécanismes conduisant aux résultats thérapeutiques observés. Enfin, ces différentes techniques ne permettent pas une élimination spécifique.

On a donc pensé à avoir recours à l'utilisation de techniques d'immunoadsorption permettant l'élimination spécifique des substances indésirables présentes dans le sang, telles que notamment les anticorps.

De nombreuses techniques de séparation par adsorption biospécifique se sont développées récemment. Selon ces techniques, un ligand est retenu dans une phase solide (matrice) et mis en contact avec un mélange dans lequel se trouve un composé à séparer, présentant de l'affinité pour le ligand immobilisé. Un complexe se forme alors et ainsi la phase solide retient le composé à séparer. On peut ainsi soit séparer les constituants d'un mélange, soit épurer un milieu complexe en retenant sélectivement un de ses constituants.

On a déjà tenté d'éliminer spécifiquement des anticorps, notamment en circulation extracorporelle sur des animaux de laboratoire, voire sur l'homme.

Dans la plupart des cas on a eu recours à des immunoadsorbants particulaires placés par exemple dans des colonnes de verre. Ces immunoadsorbants sont constitués par exemple d'antigènes fixés sur des particules de cellulose, elles-mêmes "piégées" dans de l'agarose, des antigènes retenus sur du collodion adhérent à des particules de charbon de bois ou encore des antigènes fixés sur des microsphères de nylon ou sur de la silice cristalline. Pour rendre ces différents systèmes relativement efficaces, il est, en général, nécessaire de les associer à un séparateur continu plasma/cellules sanguines et de reconstituer le sang après passage du plasma sur l'immunoadsorbant.

Malgré la complexité de ces techniques, les taux d'anticorps éliminés restent faibles. Au surplus, l'utilisation de ces matériaux particulaires présente l'inconvénient d'un risque d'érosion pouvant entraîner le passage dans le sang de particules indésirables porteuses de substances antigèniques notamment, en particulier dans le cas d'une purification du sang "in vivo" par circulation extracorporelle.

Ainsi donc, aucune des techniques déjà proposées ne permet de réaliser sans risque et avec une bonne efficacité l'épuration du sang, notamment en circulation extracorporelle.

Les recherches effectuées par l'organisme demandeur avaient donc pour but de mettre au point un dispositif ne comportant pas les inconvénients des dispositifs de l'art antérieur, c'est-à-dire en particulier un dispositif comportant un système d'adsorption de la substance indésirable à éliminer capable, sous un faible volume, de fixer une quantité importante de substance à éliminer par mise en contact avec du sang complet et ne présentant pas les inconvénients inhérents à l'utilisation d'adsorbants particulaires tels que notamment propriétés mécaniques ne permettant pas de réaliser des bioréacteurs de taille relativement importante (tassement), risques d'érosion mentionnés ci-dessus, phénomènes de coagulation.

L'invention résultant de ces recherches permet, au moins en partie, d'atteindre ce but.

Ainsi, les recherches effectuées ont permis de constater qu'il est possible de remédier, au moins en partie, aux inconvénients de l'art antérieur exposés ci-dessus grâce à un dispositif comportant un adsorbant non particulaire sur lequel est fixé par covalence un ligand sélectif pour la substance à éliminer.

Plus précisément, l'invention a pour objet un dispositif pour l'élimination ou la récupération d'un anticorps ou d'un antigène à partir du sang "in vitro" ou par circulation extracorporelle "in vivo", caractérisé en ce qu'il est essentiellement constitué d'un faisceau de fibres creuses capillaires à l'intérieur desquelles est fixé par covalence un ligand sélectif pour la substance, anticorps ou antigène, à éliminer ou récupérer, présente dans le sang.

On entend ici, par fibres capillaires, des fibres dont le diamètre intérieur est très faible, essentiellement inférieur à un millimètre.

Les fibres capillaires utilisées selon l'invention sont essentiellement constituées d'une matière support présentant une bonne inertie chimique, un grand nombre de fonctions modifiables en vue de l'activation dans des conditions douces et peu sujettes à des interactions non spécifiques.

Parmi les matières répondant à cette définition, on peut citer certains polyalcools, polyamides et polysaccharides tels que notamment l'alcool polyvinylique, les résines synthétiques obtenues par condensation d'un di-acide avec une diamine, connues sous l'appellation de nylon, et, en particulier, la cellulose régénérée ou des copolymères d'alcool éthylène-vinylique.

A l'intérieur des fibres est fixé, par covalence, un ligand spécifique de la substance à récupérer ou éliminer à savoir par exemple lorsque la substance est un anticorps, l'antigène correspondant et lorsque la substance est un antigène, l'anticorps correspondant.

En règle générale, le ligand est fixé par covalence sur la matière support, après activation de celle-ci.

L'activation des matières support portant des fonctions hydroxyle peut notamment être effectuée au moyen de bromure de cyanogène, de trichloro-s-triazine, de benzoquinone, d'un bis-époxyranne, d'épichlorhydrine ou de divinylsulfone, suivant le ligand à fixer.

L'activation des matières support comportant des fonctions amide peut être réalisée, après hydrolyse, notamment au moyen de glutaraldéhyde, d'hydrazine ou de carbodiimide.

Les fibres creuses constituées essentiellement de la matière support choisie sont rassemblées en un faisceau à l'intérieur d'un logement et le sang circule à l'intérieur de ces fibres creuses.

Selon un mode préféré de réalisation de l'invention, les fibres creuses capillaires ont des diamètres internes (diamètres utiles) de 200 à 300 μ.

- 5 -

Selon un mode tout particulièrement préféré de réalisation, on utilise en tant que support un faisceau de tubes capillaires commercialisé à titre de rein artificiel, tel que par exemple celui de la Société CORDIS INC. Corporation portant la dénomination C-DAK 1.3 (surface interne : 1,3 m$^2$) ou C-DAK 0.6 (surface interne : 0,6 m$^2$) en cellulose régénérée ou celui de la société KURARAY Co. LTD, portant la dénomination KF-101 (surface interne : 1,5 m$^2$) en copolymère d'alcool éthylène-vinylique.

Le dispositif selon l'invention permet, grâce à un choix judicieux du ligand et du matériau support, ainsi que de son mode d'activation qui est, pour un matériau et un ligand donnés, connu de l'homme du métier, d'éliminer ou extraire du sang des anticorps et antigènes variés, tant "in vitro" que "in vivo" par circulation extracorporelle.

Parmi les applications possibles, on pourra citer entre autres :

1. l'élimination spécifique d'anticorps :

Des auto-anticorps sont responsables de lésions et leur élimination au moyen du dispositif selon l'invention peut constituer une thérapeutique dépourvue des inconvénients des méthodes employées jusqu'à présent, en particulier les thérapeutiques de soustraction plasmatique. On peut citer dans ce domaine des maladies telles que le lupus érythémateux disséminé (présence d'anticorps anti-ADN et de complexes anti-ADN/ADN), le syndrôme de Goodpasture (présence d'anticorps anti-membrane basale du glomérule), et surtout la myasthénie. Dans cette dernière maladie, la responsabilité des auto-anticorps anti-récepteurs de l'acétylcholine a été démontrée et les thérapeutiques de soustraction plasmatique ont permis d'observer une corrélation étroite entre l'amélioration clinique et la diminution du titre en anticorps.

Dans un certain nombre d'autres cas, la présence d'anticorps peut se révéler néfaste pendant certaines périodes particulières et il serait utile de maintenir leur taux à une valeur basse :

- un certain nombre d'hémophiles, polytransfusés, développent des anticorps anti-facteur VIII (le facteur VIII est la glycoprotéine qui normalise la coagulation dans l'hémophilie A) et, de ce fait, peuvent subir lors d'une transfusion ultérieure un choc anaphylactique mortel. L'élimination temporaire de ce type d'anticorps au moyen du dispositif selon l'invention permettrait les transfusions sans risque d'accident ;

- dans des cas d'immunisation anti-Rh sévère chez des femmes enceintes, la possibilité d'éliminer ces anticorps anti-Rh pendant une période où ils sont très dangereux pour le foetus, serait particulièrement intéressante ;

- dans le cas de greffes de moëlle chez des receveurs incompatibles dans le système ABO, on peut envisager d'éliminer les anticorps dirigés contre ces antigènes au moyen du dispositif selon l'invention, en utilisant des stromas globulaires.

2. l'élimination spécifique d'antigènes :

Dans certains cas, il peut être nécessaire d'éliminer un antigène. On fixe alors sur le support, en tant que ligand, l'anticorps, notamment monoclonal, correspondant. Cette technique peut s'appliquer par exemple dans les hypercholestérolémies génératrices d'athérosclérose précoce, où la fréquence et l'intensité des complications cardiovasculaires au cours des formes les plus graves nécessitent des traitements plus efficaces que le régime ou les médicaments et moins aléatoires que certaines autres thérapeutiques connues (anastomose portocave, plasmaphérèse, etc...).

3. Au niveau industriel notamment, la récupération, à partir du plasma de donneurs immunisés, de certains anticorps spécifiques difficilement accessibles par d'autres méthodes que l'immuno-adsorption.

Les ligands utilisés selon l'invention peuvent être de type protéique : antigène, anticorps, comme indiqué ci-dessus, mais également d'autres types, par exemple des acides nucléiques ou des polysaccharides.

L'invention vise par conséquent, en outre un procédé pour débarrasser le sang d'un anticorps ou d'un antigène gênant "in vitro" ou par circulation extracorporelle "in vivo", caractérisé en ce qu'il consiste essentiellement à faire passer le sang à purifier à travers un dispositif selon l'invention comportant dans sa partie efficace un ligand spécifique pour la substance, anticorps ou antigène, à éliminer.

Elle vise également un procédé pour extraire du sang un anticorps ou un antigène, caractérisé en ce qu'il consiste essentiellement à faire passer du sang renfermant ledit anticorps ou ledit antigène à travers un dispositif selon l'invention, comportant dans sa partie efficace un ligand spécifique pour la substance, anticorps ou antigène, recherchée et à séparer ensuite, par des moyens connus, ladite substance fixée sur le dispositif par l'intermédiaire dudit ligand.

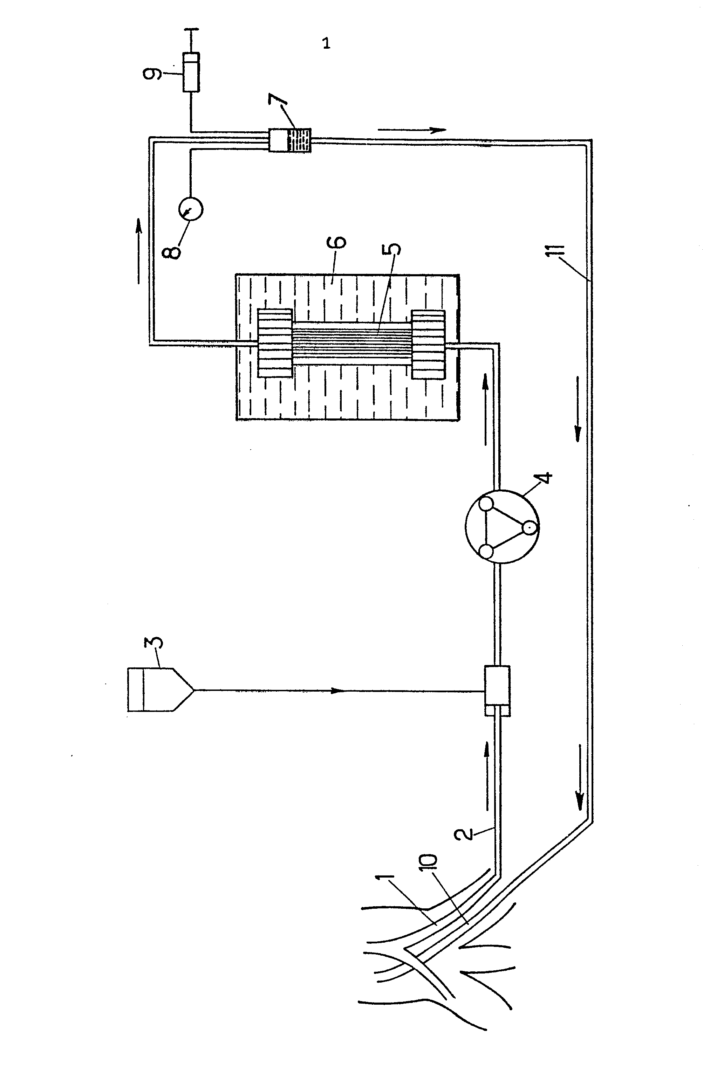

La figure unique annexée représente le schéma de principe d'un mode de réalisation du procédé de purification du sang "in vivo" par circulation extracorporelle, selon l'invention.

Le sang à purifier, prélevé dans l'artère fémorale 1 d'un animal, est envoyé dans une "ligne artérielle" 2. Un dispositif 3 permet d'ajouter une solution d'héparine dans le sérum physiologique au sang circulant dans la "ligne fémorale". Ce sang, après avoir traversé une pompe péristaltique 4, traverse le dispositif selon l'invention (bioréacteur) 5 immergé dans un bain-marie à 40°C 6. A la sortie du bioréacteur 5, le sang traverse un piège à bulles 7 destiné à le débarrasser des gaz éventuellement présents et muni d'un manomètre 8 et d'une seringue 9 destinée au contrôle du niveau. A la sortie du piège à bulles 7, le sang est renvoyé dans la veine fémorale 10 de l'animal selon la "ligne veineuse" 11.

L'invention vise en outre l'ébauche du dispositif selon l'invention telle que résultant de l'activation des fibres creuses capillaires choisies, selon un procédé spécifique du matériau constituant les fibres et du ligand devant être fixé ultérieurement par covalence.

L'invention vise tout particulièrement une telle ébauche susceptible d'être conservée pendant une période prolongée, possédant des fonctions activées stables pouvant fixer à la demande, au moment de l'emploi, différents types de ligands, antigènes ou anticorps.

L'invention sera mieux comprise à l'aide des exemples non limitatifs qui suivent.

Exemples

Dans les différents exemples, pour des raisons de meilleure accessibilité, on a utilisé, en tant que faisceaux de tubes capillaires, des modules constitués de faisceaux de tubes cappillaires commercialisés à titre de "reins artificiels", à savoir des modules en cellulose commercialisés par la société CORDIS DOW Corporation, sous les références C-DAK 1.3 et C-DAK 0.6 ayant respectivement des surfaces internes de 1,3 m$^2$ et 0,6 m$^2$ et des modules en copolymère d'alcool éthylène-vinylique commercialisés par la société KURARAY Co. Ltd sous la référence KF-101 et ayant une surface interne de 1,5 m$^2$.

1. Elimination d'anticorps (le ligand est l'antigène correspondant).

Choix des antigènes

Deux types d'antigènes de nature proétique ont été utilisés :

- des protéines purifiées solubles dans les milieux physiologiques : albumine d'une part et immunoglobulines d'autre part. L'utilisation des immunoglobulines présente un avantage supplémentaire. En effet, si ces protéines peuvent être considérées comme des antigènes vis-à-vis d'une espèce étrangère, ce sont avant tout des anticorps. L'obtention de bioréacteurs ayant fixé ces protéines est donc d'un intérêt capital si on envisage d'appliquer ces techniques à l'élimination spécifique d'antigènes.

- des protéines complexes insolubles : les expériences ont été réalisées avec des stromas d'érythrocytes (membranes de globules rouges) humains ayant conservé les susbtances antigéniques de groupe sanguin A ou B.

Préparation des "bioréacteurs", utilisation et résultats :

1.1. Activation à l'aide de BrCN (bromure de cyanogène)

Cas des antigènes solubles

Les fonctions hydroxyle de la cellulose ou de l'alcool éthylène-vinylique ont été activées par la technique classique au bromure de cyanogène. L'activation a été réalisée à l'intérieur et à l'extérieur des fibres (parce que cela est techniquement plus facile à réaliser, mais il aurait suffi qu'elle ne le soit qu'à l'intérieur), par circulation d'une solution à 10 % de BrCN maintenue à pH 11,5 par NaOH 10 N. Après activation, toute trace de BrCN a été éliminée par lavage intensif à l'eau distillée puis avec du tampon bicarbonate de sodium 0,1 M, pH 9.

Une solution protéique (20 mg/ml en tampon bicarbonate de sodium 0,1 M, pH 9) a été ensuite introduite par circulation à l'intérieur des fibres pendant 12 heures. Après nouveau lavage pour éliminer toute trace de protéine, les fonctions actives restantes ont été bloquées (à l'intérieur et à l'extérieur des fibres) par circulation d'une solution de lysine.

Cas des antigènes insolubles

Le procédé d'activation est le même que précédemment. Les protéines qui ont été fixées dans les mêmes conditions ont été utilisées en suspension fine, c'est-à-dire en suspension de microparticules obtenues par broyage de particules antigéniques insolubles, par ultrasonification.

Résultats

Modules constitués de tubes capillaires en cellulose.

Taux de fixation : Quelle que soit la nature de la protéine, des quantités de 150 à 200 mg et 300 à 500 mg

- 10 -

de protéines sont fixées à l'intérieur des fibres, respectivement pour les modules de surface interne 0,6 $m^2$ et 1,3 $m^2$.

<u>Contrôle de la fixation covalente des protéines :</u>
Ces systèmes étant destinés notamment à l'élimination "ex vivo" d'anticorps, il fallait vérifier que des antigènes (protéines fixées) n'étaient pas relargués au cours des différentes manipulations qui suivent la fixation. Des essais de fixation ont donc été réalisés à l'aide de protéines (immunoglobulines humaines) marquées à $[^{125}I]$. On a pu établir qu'aucune trace de radioactivité ne se retrouvait dans les solutions ayant circulé dans le module, après les étapes successives de fixation des protéines marquées.

De plus, des études en circulation extracorporelle, "in vivo", chez le chien, pendant 2 et 5 heures respectivement, ont montré qu'aucune radioactivité ne se retrouve au niveau des organes suivants : sang, rein, foie et thyroïde.

<u>Contrôle de l'inocuité du dispositif :</u> Des études "in vivo" chez le chien, ont permis de vérifier l'inocuité du procédé : la numération globulaire, la formule leucocytaire et de nombreux paramètres sanguins n'ont subi aucune variation significative.

<u>Utilisation des "bioréacteurs" :</u> Des antigènes (respectivement identiques à ceux utilisés dans la préparation des modules ci-dessus) ont été injectés à des animaux (lapins et chiens). Ces animaux immunisés ont fourni le sérum ou le sang pour les essais d'adsorption "in vitro". Dans tous les essais, le taux des anticorps a été mesuré par une technique sensible d'hémagglutination d'hématies revêtues des antigènes correspondants.

a) <u>Essais "in vitro"</u> : Des expériences "in vitro" réalisées avec du sérum de lapins immunisés ont permis de démontrer la forte capacité d'adsorption des bioréacteurs, ainsi que la spécificité de cette adsorption. Du sang de lapin immunisé, prélevé sur anti-coagulant (hépa-

- 11 -

rine), a été mis à circuler à l'intérieur des modules ayant fixé l'antigène qui avait servi à immuniser l'animal. Les résultats d'adsorption ont été similaires à ceux obtenus avec du sérum. Aucun problème de coagulation n'a été enregistré.

b) Essais "in vivo" : Un circuit sanguin extracorporel comprenant une ligne artérielle, une pompe péristaltique, le bioréacteur plongé dans un bain-marie, une ligne veineuse, tel que défini plus haut, a été mis en place sur des animaux préalablement immunisés.

Les expériences préliminaires ont été réalisées chez le lapin. Mais, les plus nombreuses ont été réalisées chez le chien. Les animaux avaient un poids compris entre 12 et 20 kg, ce qui a permis une meilleure évaluation des phénomènes, dans l'optique d'une application à la thérapeutique humaine. On a, dans la plupart des cas, utilisé des bioréacteurs de surface interne 1,3 m$^2$. Les tableaux I et II suivants résument quelques uns des résultats obtenus.

Tableau I :

Adsorption des anticorps anti-albumine chez un chien immunisé par de l'albumine (2 modules sont posés successivement : avec des immunoglobulines fixées puis avec de l'albumine fixée).

| | Temps d'épuration en mn | Titre des anticorps* anti-albumine |
|---|---|---|
| | 0 | 1/1024 |
| Module avec | 15 | 1/1024 |
| immunoglobulines | 30 | 1/1024 |
| fixées | 45 | 1/1024 |
| Module avec | 60 | 1/4 |
| albumine fixée | 75 | 1/1 |
| | 90 | 1/1 |

* Le titre est défini comme étant la plus grande dilution du sérum qui, après passage de celui-ci à travers le module dans les conditions indiquées, est encore capable d'agglutiner des hématies recouvertes des antigènes correspondants.

Tableau II

Adsorption des anticorps anti-immunoglobulines et anti-albumine chez un chien immunisé par les deux antigènes.

|  | Temps d'épuration en mn | Titre des anticorps anti-immunoglobulines | Titre des anticorps anti-albumine |
|---|---|---|---|
| Module avec immunoglobines fixées | 0 | 1/128 | 1/256 |
|  | 20 | 1/32 | 1/256 |
|  | 40 | 1/2 | 1/256 |
| Module avec albumine fixée | 60 | 1/2 | 1/2 |
|  | 75 | 1/2 | 1/2 |

Ces expériences montrent :

- la forte capacité des bioréacteurs à éliminer les anticorps dont le titre est ramené à des valeurs extrêmement faibles (1/1024 à 1/1 en anti-albumine -tableau I-; 1/256 à 1/2 en anti-albumine et 1/128 à 1/2 en anti-immunoglobulines -tableau II-) ;

- la rapidité de l'adsorption : entre 30 et 40 mn de circulation extracorporelle ;

- la spécificité de l'adsorption : la pose pendant 45 mn, d'un module fixant les anti-immunoglobulines sur un animal ayant été immunisé avec de l'albumine, ne modifie pas le taux d'anticorps anti-albumine.

Module constitué de tubes capillaires en copolymère d'alcool éthylène-vinylique

A titre d'exemple un module activé au bromure de cyanogène a fixé 513 mg d'IgG. Du sérum de lapin (100 ml) contenant des anticorps anti-IgG à un titre agglutinant de 1/1024, après passage à travers ce module, a vu son titre agglutinant chuter à 1/4.

1.2. Activation à l'aide de benzoquinone

Des modules C-DAK 1.3 en cellulose ont subi le traitement suivant : lavages successifs par de l'eau distillée puis du tampon bicarbonate de sodium 0,1 M, pH 8, circulation à l'intérieur des tubes d'une solution 0,05 M de
benzoquinone dans l'éthanol à 20 % en tampon bicarbonate
de sodium 0,1 M, pH 8 pendant 1 heure, lavages successifs
par de l'éthanol à 20 % dans le tampon bicarbonate, de
l'éthanol à 50 % dans le tampon bicarbonate, de l'eau distillée, une solution de NaCl 1 M, de l'eau distillée,
du tampon bicarbonate.

Une solution protéique (20 mg/ml en tampon bicarbonate
de sodium 0,1 M pH 8) a été introduite par circulation
à l'intérieur des tubes pendant 24 heures à 4°C.

Les modules ont ensuite été lavés successivement par
de l'eau distillée, une solution de KCl 1 M en tampon
acétate de sodium 0,1 M, pH 4, une solution de KCl 1 M en
tampon phosphate de sodium 0,1 M, pH 8,5, de l'eau distillée
et une solution de NaCl 0,15 M, pH 7,2.

Résultats

Des quantités de l'ordre de 500 à 800 mg de protéines
ont été fixées à l'intérieur des fibres de cellulose pour
des modules de 1,3 m$^2$.

A titre d'exemple d'utilisation, du sérum de lapin
(100 ml) contenant des anticorps anti-albumine à un titre
de 1/8192, après passage à travers un module ayant fixé
750 mg d'albumine, a vu son titre chuter à 1/64.

1.3. Activation à l'aide de 1,4-butanediol diglycidyl-
éther

Des modules C-DAK 1.3 en cellulose ont subi le traitement suivant : lavages successifs par de l'eau distillée
puis une solution de NaOH 0,2 M pendant 90 mn, circulation
à l'intérieur des fibres de 278 ml d'une solution contenant 13 % (V/V) de 1,4-butanediol diglycidyl-éther dans
NaOH 0,2 M (les 100 premiers ml de cette solution ont
été éliminés après passage) pendant 24 heures et lavage
abondant à l'aide d'une solution de phosphate de sodium
0,1 M, pH 7,4.

Une solution protéique (20 mg/ml en tampon phosphate de sodium 0,1 M, pH 7,4) a été introduite par circulation à l'intérieur des tubes pendant 24 heures à température ambiante. Les modules ont ensuite été lavés par un tampon phosphate de sodium 0,1 M, pH 7,4.

Résultats

Des quantités moyennes de 300 mg de protéines ont été fixées à l'intérieur des fibres de cellulose pour des modules de 1,3 m$^2$.

A titre d'exemple d'utilisation, un sérum de lapin (100 ml) contenant des anticorps anti-albumine à un titre de 1/2048, après passage à travers un module ayant fixé 290 mg d'albumine, a vu son titre chuter à 1/8.

1.4. Activation à l'aide de trichloro-s-triazine

Des modules C-DAK 1.3 en cellulose ont subi le traitement suivant : lavages succesifs par de l'eau distillée, une solution de NaOH 0,15 M pendant 3 heures, de l'eau distillée, 1 l de dioxanne à 10 % en eau distillée, 1 l de dioxanne à 20 % en eau distillée, 1 l de dioxanne à 50 % en eau distillée, 500 ml de triéthylamine 0,2 M en dioxanne à 50 % en eau distillée.

200 ml d'une solution d'eau distillée contenant 125 ml de dioxanne, 5 g de trichloro-s-triazine, 7,3 ml de triéthylamine ont circulé à l'intérieur des tubes pendant 2 heures. Les modules ont ensuite été lavés successivement par 1 l de dioxanne à 50 %, 1 l de dioxanne à 20 %, 1 l de dioxanne à 10 %, 1 l de tampon phosphate de sodium 0,1 M, pH 7,4. Une solution protéique (20 mg/ml en tampon phosphate de sodium 0,1 M pH 7,4) a été introduite par circulation à l'intérieur des tubes pendant 12 heures à température ambiante. Les modules ont été lavés par un tampon phosphate de sodium 0,1 M, pH 7,4 puis une solution de NaCl 0,15 M, pH 7,2.

Résultats

Des quantités moyennes de 150 mg de protéines ont été fixées à l'intérieur des fibres de cellulose pour des modules de 1,3 m$^2$.

A titre d'exemple, un module ayant fixé 112 mg d'IgG a été utilisé "in vivo" chez un chien immunisé par des IgG. Après 30 mn de circulation extracorporelle, le titre des anticorps anti-IgG est passé de 1/128 000 à 1/1 000.

2. Elimination d'antigènes (le ligand est l'anticorps correspondant).

Des IgG ont été isolées d'un sérum de chèvre immunisée contre des apolipoprotéines B et fixées à l'intérieur de fibres creuses cellulosiques selon le procédé au BrCN décrit plus haut. Les taux de fixation ont été similaires à ceux obtenus pour la fixation d'albumine ou d'IgG humaines.

56 ml de sérum hyper-lipidémique ont été mis à circuler 2 heures à travers le bioréacteur obtenu. Le taux d'apolipoprotéines B est passé de 1,75 g/l à 1.01 g/l.

3. Exemple de préparation d'ébauche

Un module C-DAK 1.3 a été activé à la benzoquinone procédé décrit ci-dessus. Le module a été conservé 12 jours à 4°C, les tubes étant remplis d'une solution d'acétate de sodium 0,1 M, pH 4,5.

Au terme de ces 12 jours, le module a été vidé, lavé en tampon bicarbonate de sodium 0.1 M, pH 8 et a subi les traitements destinés à la fixation de protéines. Ce module a fixé 600 mg d'albumine à l'intérieur des fibres creuses cellulosiques.

## REVENDICATIONS

1. Dispositif pour l'élimination ou la récupération d'un anticorps ou d'un antigène à partir du sang "in vitro" ou par circulation extracorporelle "in vivo", caractérisé en ce qu'il est essentiellement constitué d'un faisceau de fibres creuses capillaires à l'intérieur desquelles est fixé par covalence un ligand sélectif pour la substance, anticorps ou antigène, à éliminer ou récupérer, présente dans le sang.

2. Dispositif selon la revendication 1, caractérisé en ce que les fibres capillaires sont essentiellement constituées d'une matière support choisie dans le groupe constitué par les polyalcools, les polyamides et les polysaccharides et présentant une bonne inertie chimique, un grand nombre de fonctions modifiables en vue de l'activation dans des conditions douces et peu sujettes à des interactions non spécifiques.

3. Dispositif selon la revendication 2, caractérisé en ce que les fibres capillaires sont essentiellement constituées d'une matière support choisie parmi l'alcool polyvinylique, le nylon et de préférence la cellulose régénérée ou des copolymères d'alcool éthylène-vinylique.

4. Dispositif selon la revendication 3, caractérisé en ce que les fibres capillaires, en cellulose régénérée ou en copolymère d'alcool éthylène-vinylique, sont celles d'un dispositif commercialisé à titre de rein artificiel.

5. Dispositif pour l'élimination ou la récupération d'un anticorps à partir du sang "in vitro" ou par circulation extracorporelle "in vivo", caractérisé par le fait qu'il est constitué par un dispositif selon l'une quelconque des revendications 1 à 4 dans lequel le ligand fixé par covalence à l'intérieur des fibres capillaires est l'antigène correspondant à l'anticorps à éliminer ou récupérer.

6. Dispositif pour l'élimination ou la récupération d'un antigène à partir du sang "in vitro" ou par circulation extracorporelle "in vivo", caractérisé par le fait qu'il est constitué par un dispositif selon l'une quelconque des revendications 1 à 4 dans lequel le ligand

- 17 -

**0108658**

fixé par covalence à l'intérieur des fibres capillaires est l'anticorps correspondant à l'antigène à éliminer ou récupérer.

7. Procédé pour l'obtention du dispositif selon l'une des revendications 1 à 6, caractérisé en ce qu'il consiste essentiellement à soumettre un faisceau de fibres creuses capillaires, de préférence rassemblées à l'intérieur d'un logement, à une activation spécifique du matériau constituant les fibres et du ligand devant être fixé par covalence, puis à fixer ensuite le ligand par covalence à l'intérieur des fibres.

8. Ebauche du dispositif selon l'une des revendications 1 à 6, caractérisée en ce qu'elle est essentiellement constituée d'un faisceau de fibres creuses capillaires activées selon un procédé spécifique du matériau constituant les fibres et du ligand devant être fixé par covalence.

9. Procédé pour débarrasser le sang d'un anticorps ou d'un antigène gênant "in vitro" ou par circulation extracorporelle "in vivo", caractérisé en ce qu'il consiste essentiellement à faire passer le sang à purifier à travers le dispositif selon l'une des revendications 1 à 6 comportant dans sa partie efficace un ligand spécifique pour la substance, anticorps ou antigène, à éliminer.

10. Procédé pour extraire du sang un anticorps ou un antigène, caractérisé en ce qu'il consiste essentiellement à faire passer du sang renfermant ledit anticorps ou ledit antigène à travers un dispositif selon l'une des revendications 1 à 6 comportant dans sa partie efficace le ligand spécifique pour la substance, anticorps ou antigène, recherchée et à séparer ensuite ladite substance fixée sur le dispositif par l'intermédiaire dudit ligand.

Office européen
des brevets

## RAPPORT DE RECHERCHE EUROPEENNE

Numéro de la demande

EP  83 40 1872

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| X | EP-A-0 034 304  (SORINI)<br><br>* En entier, en particulier le résumé, page 1, lignes 1-3; page 13, lignes 6-11; revendication 1 * | 1-5,7-9 | A 61 M    1/03<br>A 61 K   35/14 |
| Y | | 6,10 | |
| | --- | | |
| Y | WO-A-8 002 805  (GAMBRO)<br>* En entier, en particulier page 1, lignes 8-25; page 4, lignes 29-34; page 5, lignes 5-10; page 6, lignes 25-26; figure 2 * | 6,10 | |
| A | | 2,3 | |
| | --- | | **DOMAINES TECHNIQUES RECHERCHES (Int. Cl. ³)** |
| A | DE-A-2 532 883  (KOWNATZKI)<br>* Page 3, ligne 30 - page 4, ligne 34 * | 1-10 | A 61 M |
| | --- | | |
| P,X | EP-A-0 082 345  (CORDIS)<br>* En entier, en particulier page 4, ligne 12 - page 5, ligne 35; page 7, lignes 28-31 * | 1,5-9 | |
| | --- | | |
| A | US-A-3 228 876  (DOW)<br>* Colonne 10, ligne 63 - colonne 11, ligne 20 * | 2,3 | |
| | ---                    -/- | | |

Le présent rapport de recherche a été établi pour toutes les revendications

| Lieu de la recherche<br>LA HAYE | Date d'achèvement de la recherche<br>30-12-1983 | Examinateur<br>PETZUCH M. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

OEB Form 1503. 03.82

| | DOCUMENTS CONSIDERES COMME PERTINENTS | | Page 2 | |
|---|---|---|---|---|
| Catégorie | Citation du document avec indication, en cas de besoin, des parties pertinentes | Revendication concernée | CLASSEMENT DE LA DEMANDE (Int. Cl. ³) | |
| A | US-A-4 231 877 (KURARAY)<br>* Colonne 3, lignes 16-22 *<br><br>----- | 3,4 | | |

DOMAINES TECHNIQUES
RECHERCHES (Int. Cl. ³)

Le présent rapport de recherche a été établi pour toutes les revendications

| Lieu de la recherche<br>LA HAYE | Date d'achèvement de la recherche<br>30-12-1983 | Examinateur<br>PETZUCH M. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

OEB Form 1503 03.82